# FASCICULE DE BREVET EUROPEEN

(11) **EP 0 877 346 B1**
(45) Date de publication et mention de la délivrance du brevet: **12.09.2001**
(21) Numéro de dépôt: 98401081.9
(22) Date de dépôt: 04.05.1998
(51) Int. Cl.: G08B 21/00

(54) **"Dispositif de surveillance de l'activité d'une personne et/ou de détection de chute.**
Anordnung zur Überwachung der Aktivität einer Person und/oder zur Erfassung eines Sturzes
Apparatus for monitoring the activity of a person and/or detecting the falling down of a person

(30) Priorité: 06.05.1997 FR 9705547
(43) Date de publication de la demande: 11.11.1998
(73) Titulaire: CSEM Centre Suisse d'Electronique et de Microtechnique SA, 2007 Neuchâtel (CH)
(72) Inventeur: Depeursinge, Yves, 1077 Servion (CH); Krauss, Jens, 2000 Neuchatel (CH); El-Khoury, Mario, 2052 Fontainemelon (CH)
(74) Mandataire: Caron, Gérard

(56) Documents cités:
- FR-A- 2 549 262
- US-A- 4 829 285
- US-A- 4 858 622
- US-A- 5 317 305

## Description

La présente invention est relative à un dispositif de surveillance de l'activité et/ou de détection de la chute d'une personne en vue notamment de lui porter secours en cas d'un incident mettant en danger sa survie ou son état général. Un tel incident peut être par exemple une chute de la personne. Un tel dispositif est connu p.ex. du document US-A-4 829 285.

A l'heure actuelle, il existe des réseaux de surveillance de personnes, notamment de personnes âgées, composés d'une centrale de surveillance à même de faire appel à des intervenants (personnel médical qualifié, voisins, famille, etc.) en réponse à d'éventuelles alarmes pouvant provenir de personnes sous surveillance. Celles-ci se trouvant le plus souvent à leur domicile situé dans le ressort du réseau, peuvent en cas d'incident, alerter la centrale en actionnant un bouton d'alarme monté sur un boîtier qu'elles portent au poignet ou en pendentif par exemple. Le boîtier contient un générateur d'un signal d'alarme qui par l'intermédiaire d'une liaison sans fil agit sur une centrale à domicile qui transmet le signal à la centrale, le plus souvent par l'intermédiaire du réseau téléphonique publique.

Ce type de procédure d'alerte présente la difficulté de nécessiter l'intervention de la personne en situation de danger (alarme active impliquant une action du porteur) de sorte que, si la situation est grave au point de provoquer la panique de la personne, son immobilité totale, voire une perte de conscience totale, aucun signal d'alarme ne peut être émis. Par ailleurs, les systèmes existants ne comportent aucun moyen permettant de déterminer si le boîtier est réellement porté par la personne.

L'invention a pour but de proposer un dispositif du type général indiqué ci-dessus permettant de remédier aux inconvénients des dispositifs de surveillance antérieurs.

L'invention a donc pour objet un dispositif de surveillance du comportement d'une personne en vue notamment de lui porter secours en cas d'un incident mettant en danger sa survie ou son état général, ce dispositif étant destiné à être porté par la personne et comportant des moyens pour transmettre un signal de danger et/ou un signal représentatif de l'activité de la personne, à une centrale de surveillance éloignée, ce dispositif comportant
- des moyens pour engendrer des signaux d'accélération, de vitesse et de position représentatifs du comportement et/ou de l'attitude spatiale de ladite personne, ce dispositif étant caractérisé en ce qu'il comporte en plus
- des moyens pour, à partir de l'évolution dans le temps desdits signaux d'accélération, de vitesse et de position, élaborer un facteur de probabilité représentatif de la probabilité qu'une situation dangereuse soit présente, et
- des moyens pour, en fonction de la valeur dudit facteur de probabilité, déclencher un générateur d'alarme capable de provoquer la transmission dudit signal de danger à ladite centrale.

De préférence, le dispositif de surveillance comporte également des moyens pour surveiller l'activité de ladite personne.

Il résulte de ces caractéristiques que bien que l'intervention volontaire reste toujours possible, la personne surveillée peut être dispensée d'intervenir elle-même, dans le processus d'avertissement de la centrale, ce qui constitue une indéniable amélioration de sa sécurité.

Les caractéristiques énoncées ci-dessus permettent d'obtenir d'autres caractéristiques avantageuses visant à faciliter au personnel de surveillance, l'analyse à distance de la situation dangereuse. Ainsi, l'invention rend possible d'examiner de brusques changements de comportement d'une personne surveillée et ainsi de déduire très rapidement des signaux transmis à la centrale quels sont les personnels, le type d'intervention, le matériel etc. nécessaires pour secourir efficacement la personne en danger.

D'autres caractéristiques et avantages de l'invention apparaîtront au cours de la description qui va suivre, donnée uniquement à titre d'exemple et faite en se référant aux dessins annexés sur lesquels:
- la figure 1 est un schéma synoptique d'un dispositif de surveillance selon l'invention;
- la figure 2 est une courbe représentant en fonction du temps un exemple de signal composite d'accélération, de vitesse et/ou de position pouvant être relevé en cas d'une chute d'une personne surveillée.

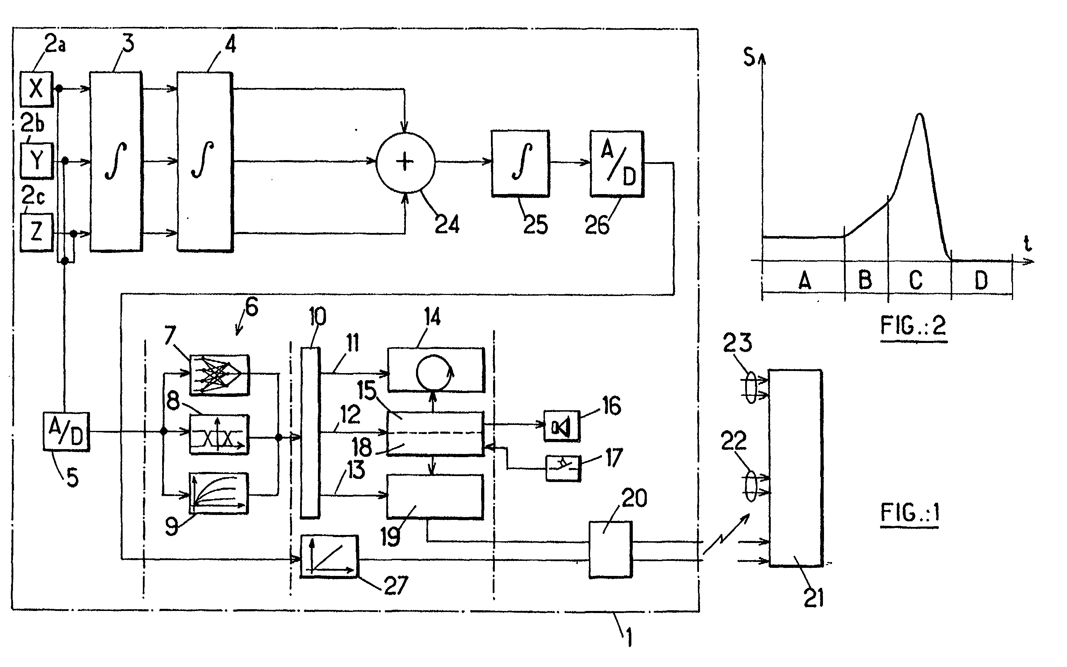

La figure 1 est un schéma simplifié d'un dispositif de surveillance du comportement d'une personne selon un mode de réalisation préféré de l'invention. Ce dispositif de surveillance, dessiné à l'intérieur du rectangle 1 de la figure 1, peut être logé dans un boîtier que la personne peut porter sur soi, par exemple sur le thorax, à l'aide d'une ceinture ou de tout autre moyen de fixation approprié.

Le dispositif de surveillance 1 comprend trois accéléromètres 2a, 2b et 2c disposés de manière à pouvoir mesurer les accélérations du boîtier dans trois directions orthogonales X, Y et Z, la direction Z étant la verticale lorsque le porteur est en station debout. Les signaux d'accélération aₓ, a_{y} et a_{z} délivrés respectivement par les accéléromètres 2a à 2c sont appliqués à deux intégrateurs successifs 3 et 4 qui fournissent respectivement les vitesses de déplacement vₓ, v_{y} et v_{z} du thorax du porteur et la position dans l'espace pₓ, p_{y}, p_{z} de ce thorax.

Les signaux engendrés à la sortie des accéléromètres 2a à 2c sont appliqués à un convertisseur analogique/digital (A/D) 5. Les signaux issus du convertisseur A/D 5 sont appliqués à un circuit de traitement de signal 6 comportant trois unités de traitement 7, 8 et 9. La première 7 de ces unités réalise une fonction d'analyse du comportement à l'aide d'un réseau neuronal artificiel examinant en permanence les signaux d'accélération, de vitesse et de position provenant du convertisseur 5. Les coefficients de poids et de polarisation de ce réseau sont déterminés par apprentissage et mis à jour au fur et à mesure de l'utilisation du dispositif de surveillance. On peut trouver une description des réseaux neuronaux utilisables à cet effet dans un article de A. Jain, J. Mao et K. Mohuiddin paru dans le n° de mars 1996 de la revue IEEE.

L'unité de traitement 8 est destinée à analyser les signaux de manière à prendre en compte le comportement du porteur avant et après un événement donnant lieu à une procédure d'alarme.

La figure 2 montre un diagramme typique en fonction du temps du signal avant numérisation dans le convertisseur 5, puis soumis à analyse pour donner lieu à une situation d'alarme. On suppose que la personne a un comportement habituel pendant une première phase A pendant laquelle le signal significatif est sensiblement constant. Pendant la phase B, on suppose que le porteur amorce une chute qui durant la phase C conduit à un choc dû à la chute. La phase D montre que le signal significatif est à zéro, le porteur étant immobile et probablement inconscient ou, tout au moins, incapable de réagir ou de se relever.

L'unité de traitement 8 examine en fonction du signal significatif pendant les phases A et D, comment il convient d'interpréter l'allure du signal pendant les phases B et C.

L'unité de traitement 9 permet d'analyser les mouvements du porteur afin d'en déduire les conditions devant donner lieu à une alarme sur le plan du comportement dynamique du porteur.

Les signaux élaborés par les unités 7, 8 et 9 sont appliqués à un circuit d'évaluation 10 destiné à évaluer en fonction de ces signaux, la probabilité qu'un événement se soit produit risquant de mettre en danger le porteur. Ce circuit est capable d'élaborer un facteur de probabilité de danger dont trois niveaux sont retenus, à savoir des niveaux de faible, moyenne ou forte probabilité, respectivement sur trois sorties 11, 12 et 13 que comporte ce circuit.

Tant qu'un signal de faible probabilité est présent sur la sortie 11, une boucle d'attente 14 est active, en attendant que le niveau du facteur de probabilité change. Lorsque la sortie 12 se présente par suite de l'apparition d'un niveau moyen du facteur de probabilité, un générateur 15 d'un signal d'avertissement est transmis à un haut-parleur 16 incorporé dans le boîtier du dispositif de surveillance 1. Le son produit par ce haut-parleur 16 est une invitation au porteur de faire connaître son état de conscience. S'il en est capable malgré la constatation d'un niveau moyen de probabilité de danger, le porteur actionne un bouton 17 se trouvant sur le boîtier du dispositif de surveillance 1. Le signal ainsi engendré est pris en compte dans un circuit de commande 18 qui transmet un signal de commande à la boucle d'attente 14 pour que cette dernière s'active de nouveau.

Si au contraire, le bouton 17 n'est pas actionné, le circuit de commande transmet un signal à un générateur d'alarme 19. Ce dernier est relié à un circuit de transmission 20 capable de mettre en forme le signal d'alarme pour l'émission, par tout moyen de transmission approprié, vers une centrale d'alarme 21 ou encore de déclencher un appel pour le personnel d'intervention. Cette centrale peut donc recevoir des informations semblables d'autres dispositifs de surveillance répartis dans le réseau desservi par la centrale 21, comme le symbolisent les connexions 22 et 23 sur la figure 1.

Avantageusement, les signaux issus de l'intégrateur 4 sont appliqués à un sommateur 24 préparant un signal d'analyse d'activité du porteur, ce signal étant appliqué à un intégrateur 25 l'intégrant sur une période de temps prédéterminée, une minute par exemple. Après conversion dans un convertisseur analogique/digital 26, le signal est transmis à une unité d'analyse d'activité 27 qui, en fonction de critères préétablis, envoie par l'intermédiaire du circuit de transmission 20 un signal d'activité à la centrale 21.

Il est généralement possible, soit de transmettre à la centrale externe les données relatives à l'activité pour un traitement éloigné, soit de les traiter localement à l'aide de circuits appropriés. On peut dès lors envisager différentes applications, telles que contrôle de régime, de temps de repos etc.

Bien entendu, le porteur peut aussi déclencher consciemment l'alarme dans le générateur d'alarme 19 par l'intermédiaire du circuit de commande 18, en pressant le bouton 17 de sa propre initiative.

L'analyse des signaux d'accélération fournis par les accéléromètres 2a, 2b et 2c permet de réaliser une autre fonction complémentaire intéressante. Ainsi, il peut être constaté si le dispositif de surveillance est porté ou non. Sinon, il peut engendrer une alarme transmise à la centrale 21 après un délai prédéterminé suivant la constatation de la dépose du dispositif.

Il permet, par la même occasion, de détecter si la personne a perdu connaissance voire, même, si elle ne respire plus.

Le dispositif de surveillance selon l'invention peut être réalisé à partir de circuits et/ou composants discrets ou modulaires, mais de préférence, il est mis en oeuvre à l'aide d'un microprocesseur convenablement programmé.

## Revendications

1. Dispositif de surveillance de l'activité et/ou de détection de chute d'une personne en vue notamment de lui porter secours en cas d'un incident mettant en danger sa survie ou son état général, ce dispositif étant destiné à être porté par l'utilisateur et comportant des moyens (20) pour transmettre un signal de danger à une centrale de surveillance éloignée (21), ce dispositif comportant
- des moyens (2a à 2c, 3 à 5) pour engendrer des signaux d'accélération, de vitesse et/ou de position représentatifs du comportement et/ou de l'attitude spatiale de ladite personne, ce dispositif étant **caractérisé en ce qu'**il comporte en plus
- des moyens (7 à 10) pour, à partir de l'évolution dans le temps desdits signaux d'accélération, de vitesse et/ou de position, élaborer un facteur de probabilité représentatif de la probabilité qu'une situation dangereuse soit présente; et
- des moyens (14 à 18) pour, en fonction de la valeur dudit facteur de probabilité, déclencher un générateur d'alarme (19) capable de provoquer la transmission dudit signal de danger à ladite centrale (21).

2. Dispositif de surveillance suivant la revendication 1, **caractérisé en ce que** lesdits moyens pour engendrer des signaux d'accélération, de vitesse et/ou de position comprennent au moins deux accéléromètres (2a à 2c) montés de manière à détecter les accélérations subies par au moins une partie du corps de ladite personne, et des moyens d'intégration (3, 4) pour effectuer une double intégration sur les signaux délivrés par lesdits accéléromètres.

3. Dispositif de surveillance suivant l'une quelconque des revendications 1 et 2, **caractérisé en ce que** lesdits moyens pour élaborer ledit facteur de probabilité, comprennent un réseau neuronal artificiel dont les valeurs de polarisation et de poids sont déterminées par apprentissage.

4. Dispositif suivant l'une quelconque des revendications 1 à 3, **caractérisé en ce que** lesdits moyens pour élaborer ledit facteur de probabilité comprennent en outre des moyens (8) pour évaluer le comportement de ladite personne avant et après l'incident constaté proprement dit.

5. Dispositif de surveillance suivant l'une quelconque des revendications 1 à 4, **caractérisé en ce que** lesdits moyens pour élaborer ledit facteur de probabilité comprennent des moyens (9) pour évaluer le comportement dynamique de ladite personne.

6. Dispositif de surveillance suivant l'une quelconque des revendications 1 à 5, **caractérisé en ce que** lesdits moyens pour déclencher un générateur d'alarme (19) comprennent une boucle d'attente (14) agencée pour rester activée tant que la valeur dudit facteur de probabilité reste représentative d'une situation non dangereuse pour ladite personne.

7. Dispositif suivant l'une quelconque des revendications 1 à 6, **caractérisé en ce que** lesdits moyens de déclenchement du générateur d'alarme (19) comprennent des moyens (15, 16) pour signaler à ladite personne un niveau prédéterminé atteint par la valeur dudit facteur de probabilité et des moyens (17) pour empêcher manuellement le déclenchement dudit générateur d'alarme (19) lorsque ladite personne perçoit ledit niveau.

8. Dispositif suivant la revendication 7, **caractérisé en ce que** lesdits moyens de signalisation sont formés par un haut-parleur (16) incorporé dans le boîtier du dispositif et en ce que lesdits moyens pour empêcher le déclenchement dudit générateur d'alarme (19) sont formés par un bouton de commande (17) se trouvant sur ledit boîtier.

9. Dispositif suivant l'une quelconque des revendications précédentes, **caractérisé en ce qu'**il comporte également des moyens (27) pour analyser l'activité de ladite personne et pour transmettre le résultat de cette analyse à ladite centrale (21).

## Claims

1. A device for monitoring the activity of a person and/or detecting a fall suffered by a person with a view in particular to summoning help in the event of an incident hazardous to life or limb, said device being adapted to be worn by said person and including means (20) for transmitting a danger signal to a remote monitoring center (21), said device including:
- means (2a - 2c, 3 - 5) for generating acceleration, speed and/or position signals representative of the behavior and/or the spatial attitude of said person,
- said device being **characterized in that** it further comprises
- means (7 - 10) for establishing a probability factor representative of the probability that a risk situation is present from how said acceleration, speed and position signals change with time; and
- means (14 - 18) for triggering in accordance with the value of said probability factor an alarm generator (19) capable of causing said danger signal to be transmitted to said center (21).

2. Monitoring device as claimed in claim 1, **characterized in that** said means for generating acceleration, speed and/or position signals include at least two accelerometers (2a - 2c) adapted to detect acceleration of at least a portion of the body of said person and integrator means (3, 4) for two-fold integration of signals delivered by said accelerometers.

3. Monitoring device as claimed in any one of claims 1 and 2, **characterized in that** said means for establishing said probability factor include an artificial neural network the bias and weighting values of which are determined by a learning process.

4. Device as claimed in any one of claims 1 - 3, **characterized in that** said means for establishing said probability factor further include means (8) for evaluating the behavior of said person before and after said incident.

5. The monitoring device as claimed in any one of claims 1 - 4, **characterized in that** said means for producing said probability factor include means (9) for evaluating the dynamic behavior of said person.

6. Monitoring device as claimed in any one of claims 1 - 5, **characterized in that** said means for triggering an alarm generator (19) include a wait loop (14) adapted to remain activated for as long as the value of said probability factor continues to be representative of a situation such that said person is not at risk.

7. Device as claimed in any one of claims 1 - 6, **characterized in that** said means for triggering said alarm generator (19) include signaling means (15, 16) for indicating to said person a predetermined level reached by the value of said probability factor and means (17) for manually preventing triggering of said alarm generator (19) if said person is aware of said level.

8. Device as claimed in claim 7, **characterized in that** said signaling means comprise a loudspeaker (16) incorporated in a unit housing said device and said means for preventing triggering of said alarm generator (19) are formed by a control button (17) on said unit.

9. Device as claimed in any one of the preceding claims, **characterized in that** it further comprises means (27) for analyzing the activity of said person and for transmitting the result of said analysis to said center (21).

## Patentansprüche

1. Vorrichtung zum Überwachung der Aktivität und/oder zum Detektieren eines Sturzes einer Person insbesondere zwecks Hilfeleistung im Fall eines ihr Leben oder ihren Allgemeinzustand gefährdenden Unfalles, wobei diese Vorrichtung von dem Benutzer zu tragen ist und Mittel (20) zum Übertragen eines Gefahrensignals an eine entfernte Überwachungszentrale aufweist, wobei diese Vorrichtung aufweist:
- Mittel (2a bis 2c, 3 bis 5) zum Erzeugen von Beschleunigungs-, Geschwindigkeits- und/oder Positionssignalen, die repräsentativ für das Verhalten und/oder die räumliche Haltung der Person sind, **dadurch gekennzeichnet, dass** sie ferner aufweist:
- Mittel (7 bis 10), um ausgehend von dem Verlauf der Beschleunigungs-, Geschwindigkeits- und/oder Positionssignale über der Zeit einen Wahrscheinlichkeitsfaktor zu ermitteln, der die Wahrscheinlichkeit, dass eine Gefahrensituation besteht, darstellt, und
- Mittel (14 bis 18), um in Abhängigkeit von dem Wert des Wahrscheinlichkeitsfaktors einen Alarmgenerator (19) auslöst, der in der Lage ist, die Übertragung des Gefahrensignals an die Zentrale (21) zu veranlassen.

2. Vorrichtung zum Überwachen nach Anspruch 1, **dadurch gekennzeichnet, dass** die Mittel zum Erzeugen der Beschleunigungs-, Geschwindigkeits- und/oder Positionssignale aufweisen: mindestens zwei Beschleunigungsmesser (2a bis 2c), die so angebracht sind, dass sie die Beschleunigungen, denen zumindest ein Körperteil der Person ausgesetzt ist, detektieren, und Integriermittel (3, 4) zum Durchführen einer zweifachen Integration der von den Beschleunigungsmessern abgegebenen Signale.

3. Vorrichtung zum Überwachen nach einem der Ansprüche 1 und 2, **dadurch gekennzeichnet, dass** die Mittel zum Ermitteln des Wahrscheinlichkeitsfaktors ein künstliches neuronales Netz aufweisen, dessen Polarisations- und Gewichtungswerte durch Lernen bestimmt werden.

4. Vorrichtung nach einem der Ansprüche 1 bis 3, **dadurch gekennzeichnet, dass** die Mittel zum Ermitteln des Wahrscheinlichkeitsfaktors ferner Mittel (8) zum Evaluieren des Verhaltens der Person vor und nach dem festgestellten eigentlichen Unfall aufweisen.

5. Vorrichtung zum Überwachen nach einem der Ansprüche 1 bis 4, **dadurch gekennzeichnet, dass** die Mittel zum Ermitteln des Wahrscheinlichkeitsfaktors Mittel (9) zum Evaluieren des dynamischen Verhaltens der Person aufweisen.

6. Vorrichtung zum Überwachen nach einem der Ansprüche 1 bis 5, **dadurch gekennzeichnet, dass** die Mittel zum Auslösen eines Alarmgenerators (19) eine Warteschleife (14) aufweisen, die solange aktiviert bleibt, wie der Wert des Wahrscheinlichkeitsfaktors für eine für die Person gefahrlose Situation repräsentativ bleibt.

7. Vorrichtung nach einem der Ansprüche 1 bis 6, **dadurch gekennzeichnet, dass** die Mittel zum Auslösen des Alarmgenerators (19) aufweisen: Mittel (15, 16), um der Person einen von dem Wert des Wahrscheinlichkeitsfaktors erreichten vorgegebenen Pegel zu signalisieren, und Mittel (17), um manuell das Auslösen des Alarmgenerators (19) zu verhindern, wenn die Person diesen Pegel wahrnimmt.

8. Vorrichtung nach Anspruch 7, **dadurch gekennzeichnet, dass** die Mittel zum Signalisieren von einem Lautsprecher (16) gebildet werden, der in dem Gehäuse der Vorrichtung vorgesehen ist, und dass die Mittel zum Verhindern des Auslösen des Alarmgenerators (19) von einem Steuerknopf (17) gebildet werden, der sich an dem Gehäuse befindet.

9. Vorrichtung nach einem der vorhergehenden Ansprüche, **dadurch gekennzeichnet, dass** sie gleichermaßen Mittel (27) zum Analysieren der Aktivität der Person und zum Übertragen des Ergebnisses dieser Analyse an die Zentrale (21) aufweist.
